# EUROPEAN PATENT APPLICATION

(11) **EP 4 339 882 A1**
(43) Date of publication of application: **20.03.2024**
(21) Application number: 22195648.5
(22) Date of filing: 14.09.2022
(51) Int. Cl.: G06T 7/00

(54) **SYSTEM FOR MEDICAL DATA ANALYSIS**

(71) Applicant: Siemens Healthineers AG, 91301 Forchheim (DE)
(72) Inventor: YEREBAKAN, Halid, Carmel, IN, 46033 (US); JEREBKO, Anna, Paoli, PA, 19301 (US)
(74) Representative: Horn Kleimann Waitzhofer Schmid-Dreyer Patent- und Rechtsanwälte PartG mbB

(57) **Abstract**

A system (100) for medical data analysis, comprising a tool generation unit (300) configured for automatically generating a first number of data analysis tools (301, 302) based on first medical image data (IMG1-6) and first analysis data (DL1-6) related to the first medical image data (IMG1-6).

## Description

The present invention relates to a system for medical data analysis, to a computer implemented method, to a computer program product and to a computer-readable medium.

Quantification of medical imaging findings plays a key role in healthcare decisions along a patient's pathway. Radiologists measure similar findings over a substantial number of repetitions. The measurement (annotation) tools used in this process include, for example, distance lines, segmentation masks, 3d bounding boxes, region of interest circles, or point annotations in the location of pathologies or anatomies. For example, distance lines are commonly used to measure aortic diameters, kidney lesions or lung nodules.

The standard approach to generate machine learning tools (e.g., neural networks) for radiology reading workflows is collecting datasets for a specific purpose by using manual measurement tools. Then, machine learning scientists analyze and clean the data to train a machine learning model. These models are optimized to estimate output variables from given input data. However, manual measurement is an expensive and time-consuming part of this process.

Yan, Ke, et al. ("DeepLesion: automated mining of large-scale lesion annotations and universal lesion detection with deep learning." Journal of medical imaging 5.3 (2018): 036501.) describe a method to train a universal CAD tool from routine clinical measurements. However, improving the single universal detector with ongoing clinical use may be challenging. Rare type of findings may have minimal impact on the model which could cause false negatives.

The object of the invention is therefore to provide an improved approach.

According to a first aspect, there is provided a system for medical data analysis, comprising a tool generation unit configured for automatically generating a first number of data analysis tools based on first medical image data and first analysis data related to the first medical image data.

By automatically generating a first number of tools, multiple different tools adapted to a specific purpose respectively may be created. In one example, two tools for (automatically) determining a distance line may be created automatically based on different images in the first medical image data, wherein the one tool is configured for determining a distance line to measure aortic diameters, the other tool to measure kidney lesions. The generation of the first number of tools may correspond to a training phase of the system. During the application phase of the system, the first number of tools may analyze the image data (corresponding to the second, third and fourth image data, see below) faster and/or with fewer false negatives as opposed to a single tool.

The system may be implemented in hardware and/or software on one or more physical devices. Multiple devices may be part of a network.

"Medical data" refers to data which is gathered from or in connection with patients or subjects in the diagnosis, treatment or prevention of illnesses.

Any "unit" herein, such as e.g. the tool generation unit, may be implemented in hardware and/or software.

"Automatically" means without human intervention. In particular, there is no human interaction between the step of providing the tool generation unit with first medical image data and the first analysis data and the generation of the first number of data analysis tools.

The first number is a real number equal or larger than 2.

The two or more different data analysis tools are different tools, meaning that they are adapted for a different purpose and/or they produce a different output for the same input.

The data analysis tools may be embodied, e. g., as one or more of the following: an algorithm, a neural network and a statistical method. The neural network may comprise any of a multilayer perceptron, a convolutional neural network, a Siamese network, a residual neural network or a triplet network, for example.

Training of the neural network may comprise adjusting weights and/or thresholds inside the neural network. The neural network may have more than 100 layers.

The data analysis tools, once trained or otherwise generated, are configured to automatically, i.e., without human intervention, analyze medical image data and/or analysis data related to the medical image data once applied thereto. Such medical image data and/or analysis data is referred to herein as the second or fourth image data and/or analysis data - as opposed to the first and third image data and/or analysis data which is used for training or otherwise creating the data analysis tools.

The first and/or third medical image data (or descriptors based thereon) may form the input data for training the data analysis tools and the first and/or third analysis data may form the desired output data of the data analysis tools. In other embodiments, the first and/or third medical image data and first and/or third analysis data are both forming the input data.

The first medical image data (as well as the second, third and/or fourth medical image data mentioned herein) may comprise two (2D)- or three (3D)- dimensional images. In particular, the first medical image data (as well as the second, third and/or fourth medical image data mentioned herein) may be made up of intensity values which may be arranged in 2D or 3D arrays, for example. The first medical image data (as well as the second, third and/or fourth medical image data mentioned below) may be captured by and received from a medical imaging unit, the medical imaging unit may include, for example, but not limited to, a magnetic resonance imaging device, a computer tomography device, an X-ray imaging device, an ultrasound imaging device, etc. The first medical image data (as well as the second and/or third medical image data mentioned herein) or respective images contained therein may comprise an organ or other anatomical structure. An organ is to be understood as a collection of tissue joined in a structural unit to serve a common function. The organ may be a human organ. The organ may be any one of the following, for example: intestines, skeleton, kidneys, gall bladder, liver, muscles, arteries, heart, larynx, pharynx, brain, lymph nodes, lungs, spleen bone marrow, stomach, veins, pancreas, and bladder. The first medical image data (as well as the second, third and/or fourth medical image data mentioned herein) or respective images contained therein may comprise one or more pathologies, including but not limited to: a tumor, a lesion, a cyst and/or a nodule.

The first analysis data (as well as the second, third and/or fourth analysis data mentioned herein) may include information (hereinafter termed "tool information") related to one or more of the following: a distance line, a segmentation mask, a bounding box, a region of interest (ROI), e.g. a circle, or a point annotation (hereinafter termed "tools"). The tool information may include coordinates of the tools (e.g. endpoints of a distance line), size (e.g. length of the distance line), geometry (e.g. of the bounding box) and/or text (e.g. as mentioned in the point annotation), for example. In addition, the first analysis data (as well as the second, third and/or fourth analysis data mentioned below) may include information (hereinafter referred to as "patient information") related to the type of organ and/or pathology comprised in the first medical image data (as well as the second, third and/or fourth image data) or other patient related information such as age, sex, weight, size etc.

The first and/or third analysis data may have been created manually (using, e.g., tools presented in a graphical user interface (GUI) such as a distance line draw tool in a computer program to analyze patient images, or entering the organ or pathology shown in the respective image using a keyboard or dropdown menu in the GUI - also known as manual annotation) by a radiologist analyzing the first and/or third medical image data, i.e., by a human. In another embodiment, a part of the first and/or third analysis data is created using neural networks. For example, the type of organ (label) is found using segmentation or classification (e.g., neural network) from the image data or a descriptor derived therefrom.

In one embodiment, data received by the tool generation unit comprises sets of data, each set comprising one or more images (contained in the first and/or third image data) and associated, with the one or more images, tool information and/or patient information (contained in the first and/or third analysis data). For example, "third" data does not require "second" data to be present.

First, second, third etc. as used herein has the mere purpose of differentiating between different sets of data, entities etc. No specific order of steps or the like is to be derived from this.

According to an embodiment, the system further comprises:
a selection unit for selecting at least one of the first number of data analysis tools; and
an execution unit for executing the selected at least one data analysis tool to, based on second medical image data, output second analysis data.

Advantageously, by the selection unit one or more of the (trained or otherwise generated) data analysis tools can be chosen and applied to second (new) data. This step corresponds to the application phase of the system. As explained above, the output data (second analysis data) may comprise a distance line, or more generally speaking a (physical) measurement value with respect to the second medical image data (e.g., the length of a tumor or other pathology in Millimeters or Centimeters).

According to an embodiment, the system further comprises a user interface configured for controlling the selection unit to select the at least one data analysis tool.

In this way, the user, e.g. the radiologist, may easily select the desired data analysis tool from all the data analysis tools which were previously generated in the training phase, for example. The user interface may be a GUI. This selection may be done during runtime, i.e. while the radiologist is reviewing (new) medical images.

According to an embodiment, the user interface is further configured to:
display the first, the second and/or third medical image data;
apply a user operated data analysis tool to the first and/or third medical image data to generate the first and/or third analysis data; and/or
display the first, the second and/or third analysis data.

For example, the user interface may, via a screen, display an image from the first medical image data. Then the user adds, by applying a user operated data analysis tool, a distance line (measuring a size of a tumor) and an annotation ("Lung") to the image (the display thus displaying the first analysis data). This image along with the analysis data (distance line, annotation) is sent to the tool generation unit which uses this set along with other data sets to generate (e.g. train) different data analysis tools.

In addition, the user interface may, via the same screen, display an image from the second image data. The user then selects one of the generated data analysis tools, for example, depending on the organ or pathology. The selected analysis tool then automatically generates the distance line in the image (for example, the distance line is overlayed with the image) without further user interaction. Said distance line then corresponds to second analysis data.

According to an embodiment, the system further comprises an update unit which is configured to control the tool generation unit to automatically:
generate, after the first number of data analysis tools has been generated, a second number of data analysis tools based on third medical image data and third analysis data related to the third medical image data; and/or
update the first number of data analysis tools based on third medical image data and third analysis data related to the third medical image data.

Thus, the tool generation unit, may be controlled by the update unit to either (1) create new data analysis tools based on new data or to (2) improve (e. g. training) existing (i.e. previously generated) data analysis tools using the new data. In embodiments, the update unit may selectively control the tool generation unit to do (1) or (2). The new or updated data analysis tools are then made available to radiologists for analyzing fourth medical image data, for example.

The second number is a real number equal or larger than 1.

According to an embodiment, the selection unit is configured for selecting at least one of the first and second number of data analysis tools.

Thereby, the second number of data analysis tools is added to the pool of existing data analysis tools, and can be selected therefrom.

According to an embodiment, the tool generation unit is configured to:
determine a number of clusters based on the first and/or third medical image data and/or first and/or third analysis data; and
generate a data analysis tool for each determined cluster.

For example, the clusters correspond to different pathologies (e.g. different types tumors) in the (e.g., first or third) medical image data and a distance line tool has been used to take measurements of the different tumors in each case. Thus, data analysis tools are created which are respectively configured to output a distance line for every type of tumor (corresponding to one cluster) automatically.

According to an embodiment, the tool generation unit is configured to determine the number of clusters by:
determining a descriptor for each image in the first and/or third medical image data; and
grouping the descriptors into the number of clusters.

The descriptor may be determined by sampling the corresponding image(s) using a sampling model and/or a (trained) neural network (e.g. an autoencoder). The training of the neural network occurs preferably before implementing the system. By using descriptors the amount of data may be reduced. Furthermore, by using a suitable descriptor, the key information may be extracted from the image prior to grouping.

According to an embodiment, the update unit is configured to control the tool generation unit to automatically generate the first and/or second number of data analysis tools when the number of descriptors in any one cluster exceeds a threshold value.

The threshold may be 100 or 1000, for example. In this manner, a new tool is only generated when sufficient data is available to make the tool accurate.

According to an embodiment, at least one of the first or second number of data analysis tools comprises a neural network, wherein generating the at least one data analysis tool comprises training the neural network with the first and/or third medical image data as input data and the first and/or third analysis data as desired output data.

Preferably, two or more of the first (or second) number of data analysis tools each comprise a neural network. Above-described embodiments of neural networks equally apply here.

According to an embodiment, the number of clusters is determined using a clustering algorithm, for example unsupervised learning.

One example of a clustering algorithm (unsupervised) is a K-means-algorithm. Unsupervised learning is well suited to detect patterns such as typical pathologies in image data. The number of clusters is ≥ 2, 10 or 100.

According to an embodiment, the clusters correspond to different organs, pathologies and/or measurement data or methods.

Clustering according to different pathologies is particularly helpful as in this way data analysis tools may be generated for new pathologies (e.g., Covid 19).

According to an embodiment, the system comprises at least a first and a second client device each connected to the tool generation unit via a network, the first client device comprising a first user interface and the second client device comprising a second user interface, wherein:
the first user interface is configured to apply the user operated data analysis tool to the first medical image data to generate the first analysis data; and
the second user interface is configured for controlling the selection unit to select the at least one data analysis tool after the tool generation unit has generated the first number of data analysis tools, the first medical image data and the first analysis data being received by the tool generation unit via the network.

According to a second aspect, there is provided a computer implemented method of medical data analysis, comprising automatically generating a first number of data analysis tools based on first medical image data and first analysis data related to the first medical image data.

According to an embodiment, at least one of the first number of data analysis tools is selected and executed to, based on second medical image data, output second analysis data.

According to an embodiment, the at least one of the first number of data analysis tools is selected by a user interface.

According to an embodiment, the user interface displays the first, second and/or third medical image data and/or displays the first, second and/or third analysis data.

According to an embodiment, a user operated data analysis tool is applied to the first and/or third medical image data to generate the first and/or third analysis data.

According to an embodiment, a second number of data analysis tools is generated based on third medical image data and third analysis data related to the third medical image data, and/or the first number of data analysis tools is updated based on third medical image data and third analysis data related to the third medical image data.

According to an embodiment, at least one of (or of both) the first and second number of data analysis tools is offered for selection to a user, wherein, preferably, the selected data analysis tool outputs, based on fourth image data, fourth analysis data.

According to an embodiment, a number of clusters is determined based on the first and/or third medical image data and/or, the first and/or third analysis data, and a data analysis tool is generated for each determined cluster.

According to an embodiment, the number of clusters is determined by:
determining a descriptor for each image in the first and/or third medical image data; and
grouping the descriptors into the number of clusters.

According to an embodiment, the first and/or second number of data analysis tools are automatically generated when the number of descriptors in any one cluster exceeds a threshold value.

According to an embodiment, the descriptors, or corresponding images, and/or analysis data corresponding to said descriptors or images, within one cluster are used to generate a corresponding data analysis tool.

According to an embodiment, at least one of the first or second number of data analysis tools comprises a neural network, wherein generating the at least one data analysis tool comprises training the neural network with the first and/or third medical image data or corresponding descriptors as input data and the first and/or third analysis data as desired output data; the number of clusters is determined using a clustering algorithm, for example unsupervised learning; and/or the clusters correspond to different organs, pathologies and/or measurement data or methods.

According to an embodiment,
a user operated data analysis tool is applied to the first medical image data to generate the first analysis data by a first user interface and/or first client device; and
after generating the first number of data analysis tools, at least one data analysis tool is selected from the first number of data analysis tools using a second user interface and/or second client device (and/or the first user interface and/or first client device).

Preferably, the first user interface is operated or executed by the first client device, the second user interface by the second client device. In an embodiment, the first number of data analysis tools is generated and/or stored on a server. The first and/or second device may communicate with the server through a network.

The first number of data analysis tools may be updated (as described above by adding a new tool or updating an existing tool) on the server. The update process may be controlled by the first or second user interface and/or first or second client device.

According to a third aspect, a computer program product comprising machine readable instructions, that when executed by one or more processing units cause the one or more processing units to perform method step(s) as described above.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a fourth aspect, a computer-readable medium on which program code sections of a computer program are saved, the program code sections being loadable into and/or executable in the above-described system to make the system execute the method step(s) as described above when the program code sections are executed in the system.

The features, advantages and embodiments described with respect to the first aspect equally apply to the second and following aspects, and vice versa.

"A" is to be understood as non-limiting to a single element. Rather, one or more elements may be provided, if not explicitly stated otherwise.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features, and advantages of the present invention will become apparent from the subsequent description and dependent claims, taken in conjunction with the accompanying drawings, in which:
- FIG. 1: illustrates a block diagram of a client-server architecture embodying a system for medical data analysis;
- FIG. 2: illustrates a block diagram of a data processing system embodying a device for medical data analysis;
- FIG. 3: illustrates a tool creation and update step according to an embodiment;
- FIG. 4: illustrates a clustering step during the training phase of the system according to an embodiment;
- FIG. 5: illustrates a training step in the training phase of the system according to an embodiment;
- FIG. 6: illustrates an execution step in the application phase (runtime) of the system according to an embodiment; and
- FIG. 7: illustrates a flowchart of an embodiment of a computer-implemented method for medical data analysis.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

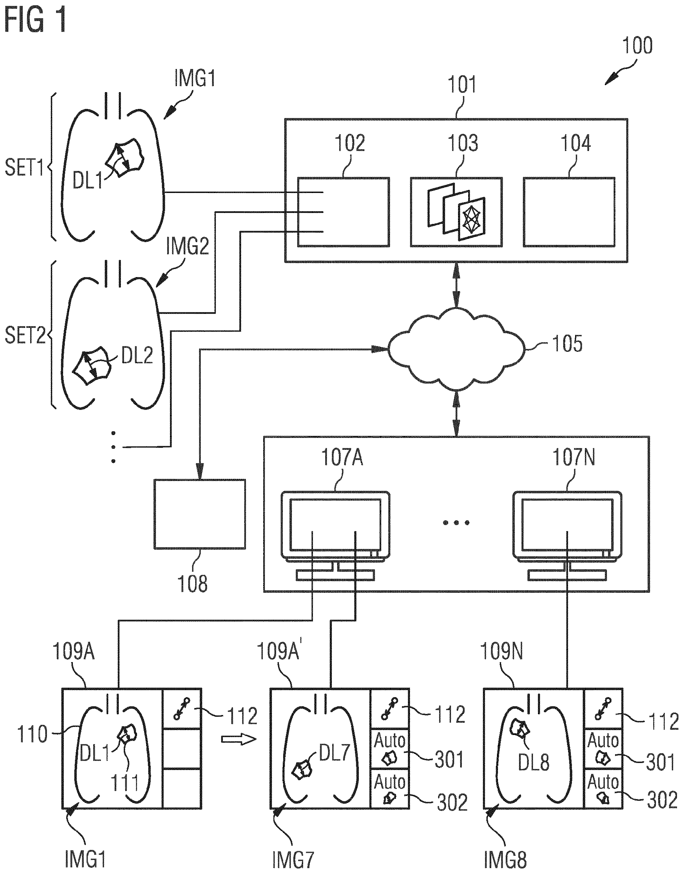

FIG. 1 provides an illustration of a block diagram of a client-server architecture 100 embodying a system for medical data analysis. The client-server architecture 100 comprises a server 101 and a plurality of client devices 107A-N. Each of the client devices 107A-N is connected to the server 101 via a network 105, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 105, for example, the internet. The cloud computing environment provides on-demand network access to a shared pool of the configurable computing physical and logical resources.

The server 101 may include a medical database 102 that comprises medical images IMG1, IMG2, etc. related to a plurality of patients as well as analysis data DL1, DL2 (in this case distance lines, for example) related to the medical images IMG1, IMG2. The medical image IMG1 and the associated distance line DL1 may form a first data set SET1, the medical IMG2 and the associated distance line DL2 may form a second data set SET2. The data sets SET1, SET2 may be associated with different patients and may have been gathered at different points in time, at different locations and/or using different client devices 107A-N. The database 102 may be maintained by a healthcare service provider such as a clinic.

The medical images IMG1, IMG2 may have been captured by an imaging unit 108. The imaging unit 108 may be connected to the server 101 through the network 105. The medical imaging unit 108 may be, for example, a scanner unit such as a magnetic resonance (MR) imaging unit, computed tomography (CT) imaging unit, an X-ray fluoroscopy imaging unit, an ultrasound imaging unit, etc.

The server 101 may include a module 103 that is configured for implementing a method for medical data analysis, in particular as described hereinafter. The module 103 may communicate with the network 105 via a network interface 104.

The client devices 107A-N are user devices, used by users, for example, medical personnel such as a radiologist, pathologist, physician, etc. In an embodiment, the user device 107A-N may be used by the user to receive medical images IMG1-8 (herein also "medical image data") associated with multiple patients. The medical image data can be accessed by the user via a graphical user interface 109A-N of an end user web application on the user devices 107A-N. In another embodiment, a request may be sent to the server 101 to access the medical images associated with the patients via the network 105.

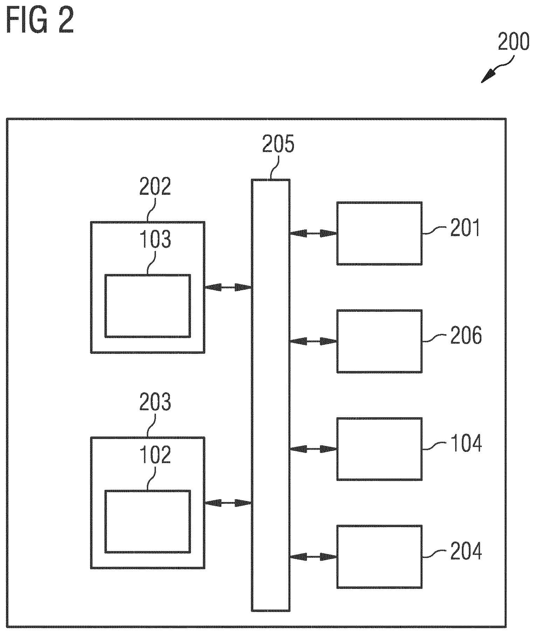

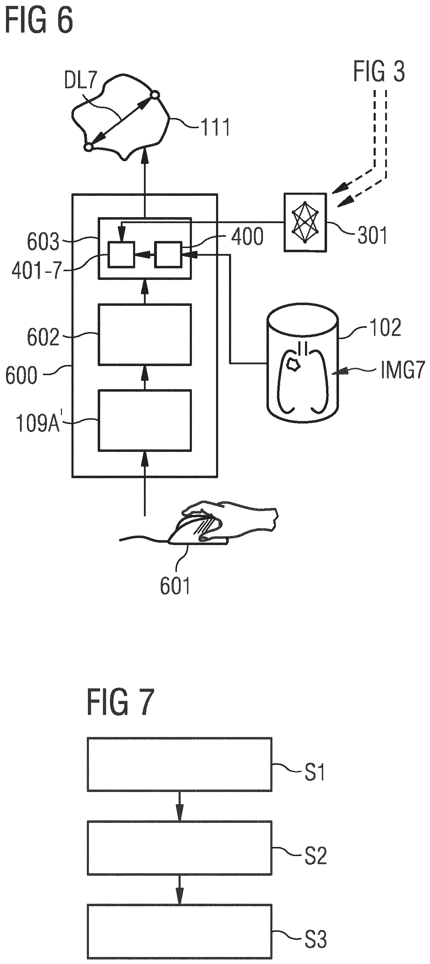

FIG. 2 is a block diagram of a data processing system 200 which, according to an embodiment, implements the server 101 of FIG. 1, the sever 101 being configured to perform one or more of the method steps (also see FIG. 7) described herein. In FIG. 2, said data processing system 200 comprises a processing unit 201, a memory 202, a storage unit 203, an input unit 204, an output unit 206, a bus 205, and the network interface 104.

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 201 may also include embedded controllers, such as generic or programmable logic devices or arrays, application specific integrated circuits, single-chip computers, and the like.

The memory 202 may be volatile memory and non-volatile memory. The memory 202 may be coupled for communication with said processing unit 201. The processing unit 201 may execute instructions and/or code stored in the memory 202. A variety of computer-readable storage media may be stored in and accessed from said memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 201 comprises a module 103 stored in the form of machine-readable instructions on any of said above-mentioned storage media and may be in communication to and executed by processing unit 201. When executed by the processing unit 201, the module 103 causes the processing unit 201 execute one or more steps of the method as elaborated upon in detail in the following figures.

The storage unit 203 may be a non-transitory storage medium which stores the medical database 102. The input unit 204 may include input means such as keypad, touch-sensitive display, camera (such as a camera receiving gesture-based inputs), a port etc. capable of providing input signal such as a mouse input signal or a camera input signal. The bus 205 acts as interconnect between the processor 201, the memory 202, the storage unit 203, the input unit 204, the output unit 206 and the network interface 104. The data sets SET1, SET2 (see FIG. 1) may be read into the medical database 102 via the network interface 104 or the input unit 204, for example.

Those of ordinary skilled in the art will appreciate that said hardware depicted in FIG. 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 200 in accordance with an embodiment of the present disclosure may comprise an operating system employing a graphical user interface (GUI). Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. Said operating system is modified or created in accordance with the present disclosure as described. Disclosed embodiments provide systems and methods for processing medical images.

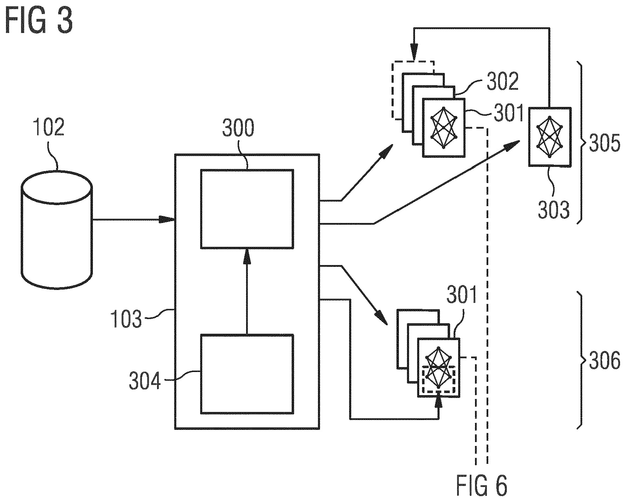

FIG. 3 shows a tool generation unit 300 which may be implemented in hardware and/or software. For example, the tool generation unit 300 is part of the module 103. The tool generation unit 300 automatically generates a (first) number of data analysis tools 301, 302 (in this case two, for example) based on the data sets SET1, SET2 (FIG. 1). For example, more than one hundred or more than one thousand data sets could be used in this. This corresponds to step S1 of Fig. 7, illustrating a computer implemented method of medical data analysis in one embodiment.

The data sets SET1, SET2 may be obtained as follows. The user (e.g. radiologist) causes the GUI 109A (FIG. 1) to display the medical image IMG1. The medical image IMG1 (also termed "first medical image data" herein) may be stored in the database 102, or may be directly obtained from the imaging unit 108. In this case, the medical image IMG1 is showing a 2D image of a human lung 110 including a tumor 111. Then the user selects a manually operated (e.g., using a mouse) data analysis tool 112 from the GUI 109A. Said tool 112 is configured to draw the distance line DL1. With the distance line DL1 the physical size of the tumor 111, e.g. its diameter or any other dimension, is measured, e.g., in mm or cm (also termed "first analysis data" herein). Instead, the tool 112 could be configured to draw a segmentation mask, a bounding box or a region of interest (ROI), e.g. a circle, or to make a point annotation. Except for the point annotation, the tools are each configured to determine a certain dimension or region of the tumor 111 by using pixel coordinates, thereby gathering some measurement information with regard to the tumor 111. Of course, instead of the tumor 111, any other pathology such as a nodule, lesion, cyst etc. could be analyzed using a suitable tool 112. This process is repeated for the medical image IMG2, giving the distance line DL2, and a plurality of other images (not shown). In particular, these images and distance lines are collected from other clients 107N, where these other clients may even be located at other clinics etc.

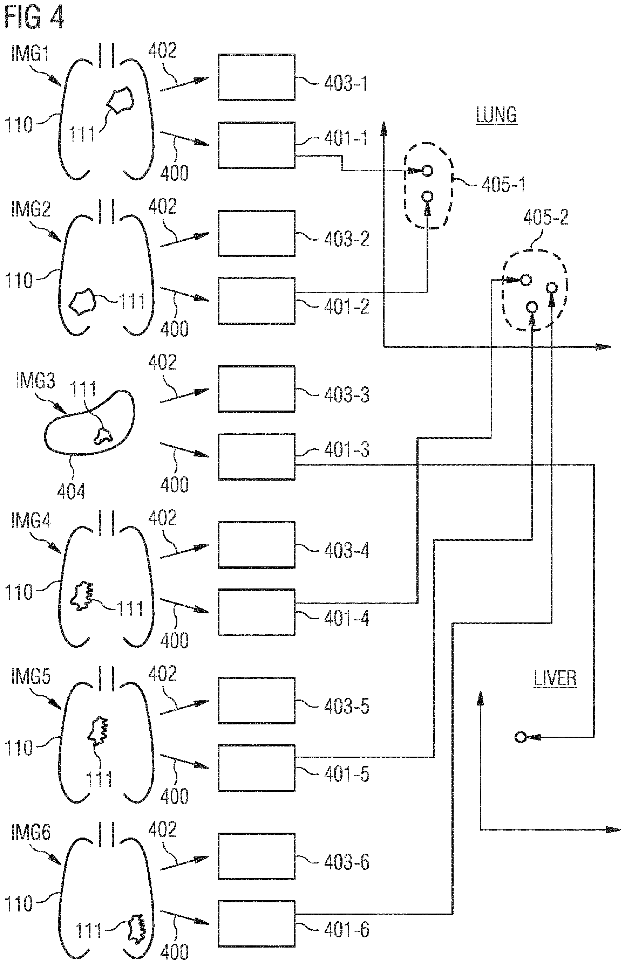

Turning now to Fig. 4, a plurality of medical images IMG1-6 are shown, each of which forming a data set with corresponding analysis data (not shown), said data said being obtained as explained for the data sets IMG1, IMG2.

To each of the images IMG1-6 the tool generation unit 300 applies an autoencoder 400 (trained neural network) to obtain a descriptor 401-1 to -6 for each image IMG1-6. Also, a segmentation or classification 402 may be applied, by the tool generation unit 300, to each image IMG1-6 to obtain a label 403-1 to -6 for each organ shown in each image IMG1-6. For segmentation or classification 402 a trained neural network or unsupervised learning may be used, for example. For example, the labels obtained by segmentation or classification 402 are "lung" except for IMG4 showing a liver 404 in which case the label 403-3 is "liver". The autoencoder 400 and/or the segmentation or classification 402 may be the same for each IMG1-6 (and for the entire tool generation process), and they may be pre-trained, meaning that they are trained prior to the implementation of the system 100. Instead of using a segmentation or classification 402, the user may provide the relevant organ label manually via the GUI 109A-N which then may be associated with each image IMG1-6 (e.g., by adding the respective label to each data set SET1, SET2 etc.).

In a further step, the descriptors 401-1 to -6 may be classified by the tool generation unit 300 in accordance with their respective annotation 403-1 to -6. As shown all descriptors 401-1,2,4,5,6 are associated with the label "lung", whereas the descriptor 401-3 is associated with the label "liver".

Furthermore, the descriptors 401-1 to -6 may be classified in accordance with the type of user operated data analysis tool 112 (distance line tool, segmentation mask tool, bounding box tool etc.) they were each obtained with (not shown).

Then, the descriptors 401-1,2,4,5,6 are classified using an unsupervised classification algorithm such as K-means. Thereby, clusters 405-1, 405-2 are identified. For example, the clusters 405-1, 405-2 may correspond to different pathologies. When comparing the tumors 111 in IMG1,2 versus the IMG3,4,5 it can be seen that they show a different type of tumor 111. The same process is followed for other descriptors (here descriptor 401-3), labels and tools 112, but not described here further.

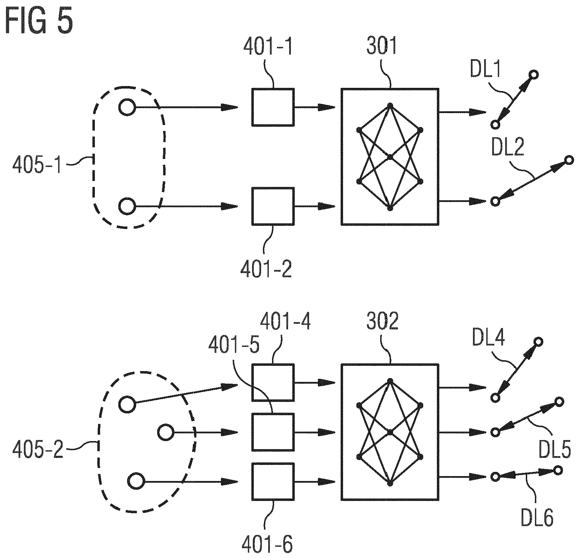

As shown in Fig. 5, a data analysis tool 301, 302 is trained for every cluster 405-1, 405-2 by the tool generation unit 300. It may be provided that such training only begins for each cluster when the number of descriptors 401-1,2,4,5,6 exceeds a threshold value of, e.g., 100. The data analysis tools 301, 302 are initially untrained neural networks, e.g., residual neural networks. The data analysis tools 301, 302 are trained using the descriptors in each cluster as input data, so for cluster 405-1 and data analysis tool 301, the input data is comprised of the descriptors 401-1 and 401-2. The output data is retrieved from the data set SET1, SET2 with which the descriptors 401-1, 401-2 or their images IMG1, IMG2 are associated with, namely the distance lines DL1, DL2. Taking the same approach, the data analysis tool 302 is trained to output distance lines DL4-6 for descriptors 401-4,5,6 as input.

With the data analysis tools 301, 302 thus automatically generated (training phase completed), these may be applied (also termed runtime or application phase herein) as indicated in step S2 of FIG. 7. Returning to FIG. 1, the GUI 109A may be automatically (or manually) updated to show that, e.g., data analysis tools 301, 302 are now available. The updated GUI 109A' offers a new selection option 301, 302 below the manual tool 112.

The updated GUI also shows a new medical image IMG7 (herein also referred to as "second medical image data") corresponding to a new patient retrieved from the database 102 or directly from the imaging unit 108. Now, instead of manually applying the distance line using the tool 112, the user can select a suitable tool 301, 302 and the distance line DL7 will be added automatically, as explained with regarding FIG. 6 in more detail. Instead, the suitable tool may be selected automatically by matching the pathology in IMG7 to one of the clusters 405-1, 405-2.

FIG. 6 shows a module 600 which may form part of the module 103 or may be a separate module running on the server 101 (processing unit 201) and/or may run on a client device 107A-N. The module 600 provides the GUI 109A'. Using a peripheral device 601 (such as a mouse as shown), the user clicks on the data analysis tool 301 (button) as part of the GUI 109A'. The GUI 109A' then controls a selection unit 602 to select, from the available data analysis tools 301, 302, the data analysis tool 301 (trained neural network) for execution by an execution unit 603. The execution unit 603 retrieves the medical image IMG7 from the data base 102 and applies the autoencoder 400 thereto to obtain a descriptor 401-7. Then, the execution unit 603 applies the data analysis tool 301 to the descriptor 401-7 to obtain the distance line DL7 for the tumor 111. The distance line DL7 comprises a measured size of the tumor, e.g., in mm or cm.

Returning to FIG. 1, the client device 107N may comprise a GUI 109N. The data analysis tools 301, 302 become also available for selection and execution (by applying the tools 301 or 302 to a new medical image IMG8 to obtain the distance line DL8) through the GUI 109N. In one embodiment, the manual tool 112 is also provided in the GUI 109N.

FIG. 3 further illustrates an update process in the further part of the application phase, corresponding to step S3 in FIG. 7. To this end, the module 103 may comprise an update unit 304. Every time a user uses the manually operated data analysis tool 112 (FIG. 1) on new images, e.g. the image IMG8 (if not automatically analyzed using the tools 301, 302, but manually using the tool 112; the image IMG8 is also termed "third medical image data" herein) new analysis data (also termed "third analysis data" herein) is generated. New descriptors are then automatically generated and classified as explained with reference to FIG. 4 above. Once sufficient descriptors have been determined, one of two processes is started, for example. Either, a new data analysis tool 303 is automatically generated and becomes available for selection via the GUI 109A-N (FIG. 1) and the selection unit 602 (FIG. 6). This will, e.g., be the case when the new descriptors are associated with a new cluster, and the number of descriptors in said cluster exceeds a certain threshold, e.g., 100. This process is labeled with reference numeral 305 in FIG. 3. Or, the new descriptors are associated with an existing cluster. Then, an existing data analysis tool, e.g., the tool 301, is further trained using these new descriptors in the existing cluster (405-1 in FIG. 5), thus obtaining an improved tool 301 and providing the same via the GUI 109A-N (FIG. 1) and the selection unit 602 (FIG. 6). In one embodiment, this process (indicated by reference numeral 306 in FIG. 3) will only start, once the number of new descriptors in the existing cluster exceeds a threshold value, e.g., 50.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein, rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

### REFRENCE SIGNS

- 100: system
- 101: computer-implemented device
- 102: medical database
- 103: module
- 104: network interface
- 105: network
- 107A - 107N: client devices
- 108: medical imaging unit
- 109A, 109A', 109N: graphical user interfaces
- 110: lung
- 111: tumor
- 112: manually operated data analysis tool
- 200: data processing system
- 201: processing unit
- 202: memory
- 203: storage unit
- 204: input unit
- 205: bus
- 206: output unit
- 300: tool generation unit
- 301, 302, 303: data analysis tools
- 304: update unit
- 305, 306: update processes
- 400: auto encoder
- 401-1 to -6: descriptors
- 402: segmentation
- 403-1 to -6: organ labels
- 404: liver
- 405-1, 2: clusters
- 600: module
- 601: mouse
- 602: selection unit
- 603: execution unit

- IMG1-IMG8: images
- DL1-8: distance lines
- SET1, SET2: data sets

- S1-S3: method steps

## Claims

1. A system (100) for medical data analysis, comprising a tool generation unit (300) configured for automatically generating a first number of data analysis tools (301, 302) based on first medical image data (IMG1-6) and first analysis data (DL1-6) related to the first medical image data (IMG1-6).

2. The system of claim 1, further comprising:
a selection unit (602) for selecting at least one of the first number of data analysis tools (301, 302); and
an execution unit (603) for executing the selected at least one data analysis tool (301, 302) to, based on second medical image data (IMG7), output second analysis data (DL7).

3. The system of claim 2, further comprising a user interface (109A-N) configured for controlling the selection unit (602) to select the at least one data analysis tool (301, 302).

4. The system of one of claims 1 - 3, wherein the user interface (109A-N) is further configured to:
display the first, the second and/or third medical image data (IMG1-6, IMG7, IMG8);
apply a user operated data analysis tool (112) to the first and/or third medical image data (IMG1-6, IMG8) to generate the first and/or third analysis data (DL1-6, DL8); and/or
display the first, the second and/or third analysis data (DL1-6, DL7, DL8).

5. The system of one of claims 1 - 4, further comprising an update unit (304) which is configured to control the tool generation unit (300) to automatically:
generate a second number of data analysis tools (303) based on third medical image data (IMG8) and third analysis data (DL8) related to the third medical image data (IMG8); and/or
update the first number of data analysis tools (301) based on third medical image data (IMG8) and third analysis data (DL8) related to the third medical image data (IMG8).

6. The system of claim 5, wherein the selection unit (602) is configured for selecting at least one of the first and second number of data analysis tools (301, 302, 303).

7. The system of one of claims 1 - 6, wherein the tool generation unit (300) is configured to:
determine a number of clusters (405-1, 405-2) based on the first and/or third medical image data (IMG1-6, IMG8) and/or first and/or third analysis data (DL1-6, DL8); and
generate a data analysis tool (301, 302) for each determined cluster (405-1, 405-2).

8. The system of claim 7, wherein the tool generation unit (300) is configured to determine the number of clusters (405-1, 405-2) by:
determining a descriptor (401-1 to -6) for each image (IMG1-6, IMG8) in the first and/or third medical image data; and
grouping the descriptors (401-1 to -6) into the number of clusters (405-1, 405-2).

9. The system of claim 8, wherein the update unit (304) is configured to control the tool generation unit (300) to automatically generate the first and/or second number of data analysis tools (301, 302, 303) when the number of descriptors (401-1 to 6) in any one cluster (405-1, 405-2) exceeds a threshold value.

10. The system of claim 8 or 9, wherein the tool generation unit (300) is configured to use the descriptors (401-1 to 6), or corresponding images (IMG1-6) and/or analysis data (DL1-6) corresponding to said images (IMG1-6), within one cluster (405-1, 405-2) to generate a corresponding data analysis tool (301, 302).

11. The system of one of claims 1 - 10, wherein:
at least one of the first or second number of data analysis tools (301, 302, 303) comprises a neural network, wherein generating the at least one data analysis tool (301, 302, 303) comprises training the neural network with the first and/or third medical image data (IMG1-6, IMG8) or corresponding descriptors (401-1 to 6) as input data and the first and/or third analysis data (DL1-6, DL8) as desired output data;
the number of clusters (405-1, 405-2) is determined using a clustering algorithm, for example unsupervised learning; and/or
the clusters (405-1, 405-2) correspond to different organs (110), pathologies (111) and/or measurement data or methods.

12. The system of one of claims 2 - 11, comprising at least a first and a second client device (107A-N) each connected to the tool generation unit (300) via a network (105), the first client device (107A) comprising a first user interface (109A) and the second client device (107N) comprising a second user interface (109N), wherein:
the first user interface (109A) is configured to apply the user operated data analysis tool (112) to the first medical image data (IMG1-6) to generate the first analysis data (DL1-6); and
the second user interface (109N) is configured for controlling the selection unit (602) to select the at least one data analysis tool (301, 302) after the tool generation unit (300) has generated the first number of data analysis tools (301, 302), the first medical image data (IMG1-6) and the first analysis data (DL1-6) being received by the tool generation unit (300) via the network (105).

13. A computer implemented method of medical data analysis, comprising automatically generating (S1) a first number of data analysis tools (301, 302) based on first medical image data (IMG1-6) and first analysis data (DL1-6) related to the first medical image data (IMG1-6).

14. A computer program product comprising machine readable instructions, that when executed by one or more processing units (201), cause the one or more processing units (201) to perform method step according to claim 13.

15. A computer readable medium on which program code sections of a computer program are saved, the program code sections being loadable into and/or executable in the system (100) of one of claims 1 - 12 to make the system (100) execute the method step according to claim 13, when the program code sections are executed in the system (100).
